# EUROPEAN PATENT SPECIFICATION

(11) **EP 2 175 917 B1**
(45) Date of publication and mention of the grant of the patent: **26.06.2013**
(21) Application number: 08788236.1
(22) Date of filing: 28.07.2008
(51) Int. Cl.: A61M 5/20, A61M 5/32

(54) **INJECTION DEVICE WITH LOCKING MECHANISM FOR SYRINGE CARRIER**
INJEKTIONSVORRICHTUNG MIT SPERRMECHANISMUS FÜR SPRITZENTRÄGER
DISPOSITIF D'INJECTION À MÉCANISME DE VERROUILLAGE POUR PORTE-SERINGUE

(30) Priority: 08.08.2007 GB 0715457
(43) Date of publication of application: 21.04.2010
(73) Proprietor: Cilag GmbH International, 6300 Zug (CH)
(72) Inventor: JENNINGS, Douglas, Ivan, Royston, Hertfordshire SG8 7XU (GB)
(74) Representative: Tunstall, Christopher Stephen
(86) International application number: PCT/GB2008/002583
(87) International publication number: WO 2009/019440

(56) References cited:
- WO-A-2005/115508
- WO-A-2007/036676
- WO-A-2007/138299
- GB-A- 2 424 836

## Description

### Field of the Invention

The present invention relates to an injection device of the type which has a syringe and which extends the syringe, discharges its contents and then retracts it automatically.

### Background of the Invention

Injection devices are shown in WO 95/35126 and EP-A-0 516 473. These devices employ a drive spring and some form of release mechanism that releases the syringe from the influence of the drive spring once its contents are supposed to have been discharged, to allow it to be retracted by a return spring.

Generally, the return spring is relatively weak, since its restoring force must be overcome by the drive spring, even while the drive spring is doing work on the various components of the injection device and the syringe during an injection cycle. This may give rise to a problem when the injection device is used with sealed hypodermic syringes, which typically have a hermetically sealed cover, needle shield or "boot" that covers the hypodermic needle and maintains the sterility of the syringe contents. Naturally, it is necessary to maintain the sterility of the syringe contents up to the point of administration, which for devices that are designed to be disposable, as many will be, means that the boot must be removed with the syringe inside the injection device.

Typically, the action required to remove the boot from the syringe is simply to pull the boot away from the syringe, which requires a force in excess of 20N. This is significantly greater than the restoring force of the return spring, so the syringe will be pulled out of the injection device as the boot is removed and, when the boot comes away, it will snap back into place. This is not the best way to handle the syringe. The shock could damage it, the needle could be damaged and there may be problems re-engaging the syringe with those components of the injection device designed to act upon it. Even in cases where there is no return spring, for example where the syringe is held in place by friction with components of the injection device, the problem will still arise of relocating the syringe onto those components of the injection device designed to act upon it.

Moreover, there is a problem with having the syringe generally moveable in a direction out of the injection device. Accidental activation of the drive spring by mechanical failure of the drive spring's release mechanism (e.g. a trigger) can occur, for example by dropping the device on a hard surface. This accidental activation could cause the syringe to be extended unintentionally out of the device and its contents to be ejected. This could expose the needle of the syringe and increase the risk of inadvertent ski puncturing and/or infection.

International publication no. WO 2007/036676 discloses an injection device having a housing that receives a syringe having a sealed boot that covers its needle. A releasable locking mechanism retains the syringe in its retracted position. A sleeve projects from the exit aperture and can be depressed to release the locking mechanism. A removable threaded cap closes the housing, covers the exit aperture and the sleeve, thus preventing the locking mechanism from being released, and engages the boot on the syringe. When the cap is removed, it takes the boot with it, no longer closes the exit aperture and no longer prevents the locking mechanism from being released. Then, the locking mechanism can be released and the injection cycle begun.

International publication no. WO 2005/115508 discloses an injection device having a housing and a housing closure means. The injection device houses a syringe having a needle which is sealed by a boot. The housing closure means is arranged so that the boot can be connected to the housing closure means simply, but cannot be removed from the housing closure means. The housing and housing closure means are arranged so that upon rotation of the housing closure means, the housing closure means is moved axially away from the housing and the boot and the boot is removed from the syringe. The injection device is simple to use and manufacture.

UK patent publication no. GB 2424836 discloses an injection device having a housing and a housing closure means. The injection device houses a syringe having a needle which is sealed by a boot. The housing closure means is arranged so that the boot can be connected to the housing closure means simply, but cannot be removed from the housing closure means. The housing and housing closure means are arranged so that upon rotation of the housing closure means, the housing closure means member first rotates relative to the housing and then moves away from the housing and removal of the housing closure member removes the boot from the syringe.

### Summary of the Invention

The injection device of the present invention is designed to deal with the aforementioned problems.

In a first aspect of the present invention, there is provided An injection device comprising:
a housing adapted to receive a syringe having a discharge nozzle, the syringe being movable along a longitudinal axis of the housing between a retracted position in which the discharge nozzle is contained within the housing and an extended position in which the discharge nozzle extends from the housing through an exit aperture;
a cap removably located over the exit aperture;
a syringe carrier adapted to support the syringe as it is advanced; and
a needle shield over the discharge nozzle,
wherein the needle shield is connected to the cap,
wherein the cap comprises a locking component which is adapted to prevent, in an engaged position when the cap is located on the housing, movement of the syringe carrier towards the exit aperture relative to the housing as the needle shield is removed from the discharge nozzle,
wherein the needle shield is moveable within the cap in a linear direction towards the exit aperture to release the needle shield from the discharge nozzle.

In this way, the syringe carrier cannot move linearly within the injection device whilst the cap is in place over the exit aperture. However, the needle shield can still be moved and released from the discharge nozzle whilst the cap is in place by rotation of the cap. The cap prevents the syringe carrier moving forward whilst the needle shield is being removed. This means that the syringe does not become damaged as the needle shield is moved into its forward position, for example by having the needle shield snap back into place over the discharge nozzle.

Preferably, the cap is rotatable on the injection device to move the needle shield in its linear direction towards the exit aperture.

Preferably, the cap comprises a body and the locking component is a sleeve located within the body and fixed relative to the body.

The cap may further comprise a shield retainer adapted to grip the needle shield.

In one embodiment of the present invention, the body of the cap is rotatable relative to the housing whilst the shield retainer does not rotate relative to the discharge nozzle, wherein the cap comprises means for converting rotational movement of the cap relative to the housing into linear movement of the shield retainer away from the exit aperture along the longitudinal axis.

Preferably, the shield retainer is located within the sleeve.

In one embodiment of the present invention, the converting means is provided by a first thread on the shield retainer and a second thread on the sleeve and the first and second threads engage each other so that rotational movement of the body of the cap results in linear movement of the shield retainer with respect to the exit aperture.

Preferably, the shield retainer is provided with a shield grip and a shield pull component connected to the shield grip, wherein the first thread is provided on the shield pull component.

The rotational movement may be rotation about the longitudinal axis. Preferably, the movement is anti-clockwise rotational movement, when viewing the cap from its end.

Advantageously, the sleeve may comprise a first abutment surface adopted to abut a second abutment surface on the syringe carrier when the cap is located over the exit aperture, thereby preventing movement of the syringe carrier along the longitudinal axis towards the exit aperture.

In a second aspect of the present invention, the syringe may be moveable along a longitudinal axis of the housing between a retracted position in which the discharge nozzle is contained within the housing and an extended position in which the discharge nozzle extends from the housing through an exit aperture, the cap gripping a needle shield of the syringe, the method comprising:
rotating the cap about the longitudinal axis such that the needle shield is moved along the longitudinal axis out of the exit aperture, thereby releasing it from the discharge nozzle whilst a locking component in the cap prevents movement of the syringe along the longitudinal axis; and
when the needle shield has been released from the discharge nozzle, moving the cap along the longitudinal axis to expose the exit aperture and release the locking component so that the discharge nozzle can be moved to its extended position.

### Brief Description of the Drawings

The invention will now be described by way of example with reference to the accompanying drawings, in which:
Fig. 1a is a right-side view of the injection device according to the present invention;
Fig. 1b is a perspective view of the injection device of Fig. 1 with its cap removed;
Fig. 1c is a perspective view of the cap of the injection device of Fig. 1;
Fig. 2a is an exploded right-side view of the injection device of Fig. 1;
Fig. 2b is a right-side view of the assembled components of the injection device of Fig. 1;
Fig. 2c is a perspective view of a multi-component drive used in the injection device of Fig. 1; and
Figs. 3a and 3b are cross-sectional views of the injection device of Fig. 1.

### Detailed Description of the Drawings

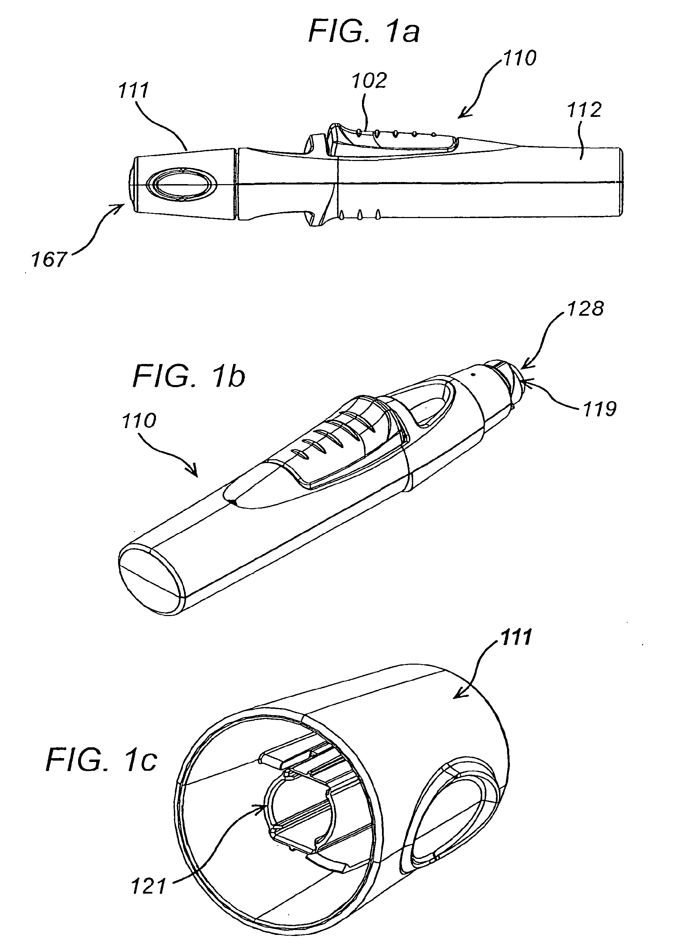

Fig. 1a is a right-side view of an injection device 110 according to the present invention. The injection device 110 has a housing 112, a cap 111 which is removable from a proximal end 167 of the housing 112 and a trigger button 102. Other parts of the device will be described in greater detail below.
Fig. 1b is a perspective view of the injection device 110 according to the present invention with the cap (not shown) removed from its end. The end of the housing 112 has an exit aperture 128, from which the end of a sleeve 119 can be seen to emerge.
Fig. 1c is a perspective view of the cap 111 of the injection device 110 according to the present invention. The cap 111 has a central boss 121 that fits within the sleeve 119 when the cap 111 is installed on the housing 112.

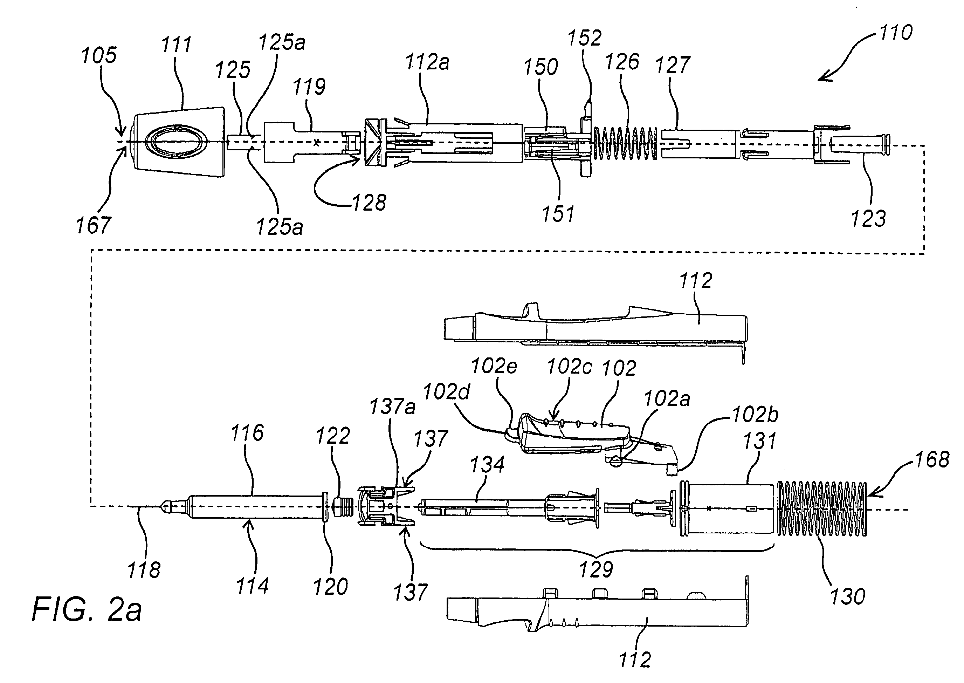

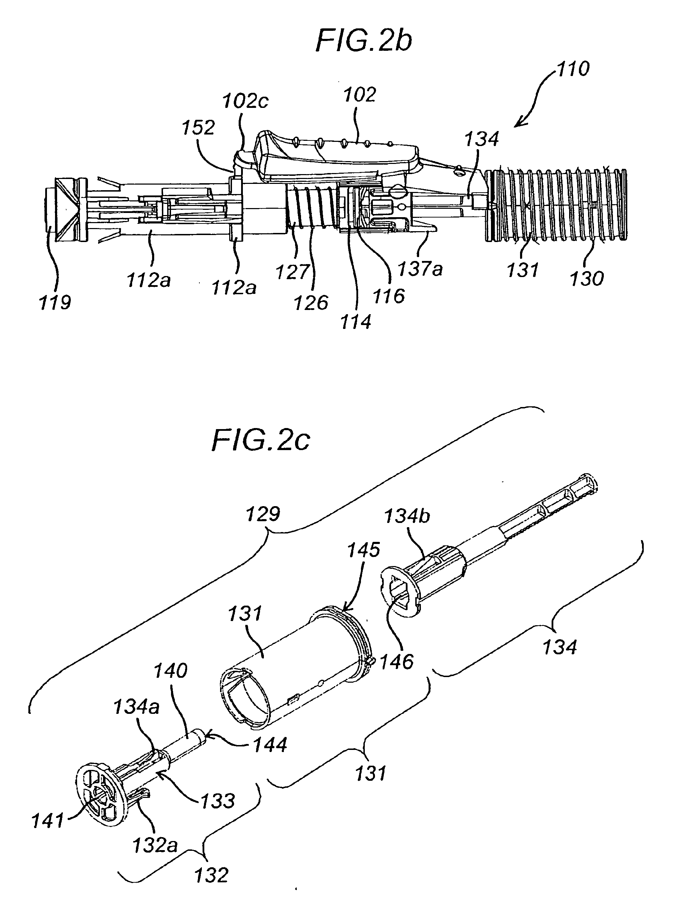

Fig. 2a is an exploded right-side view of the components of the injection device 110 according to the present invention and Fig. 2b is a right-side view of the assembled components of the injection device 110 according to the present invention without the housing 112 or cap 111.

As illustrated, the injection device 110 comprises a hypodermic syringe 114 of conventional type, including a syringe body 116 terminating at one end in a discharge nozzle, specifically a hypodermic needle 118, and at the other in a flange 120. The conventional plunger that would normally be used to discharge the contents of the syringe 114 manually has been removed and replaced with a drive element (referred to below as the second drive element 134) that contacts a bung 122 in the syringe 114. The bung 122 constrains a drug (not shown) to be administered within the syringe body 116. Whilst the syringe illustrated is of hypodermic type, this need not necessarily be so. Transcutaneous or ballistic dermal and subcutaneous syringes may also be used with the injection device of the present invention.

As illustrated, the injection device 110 includes a return spring 126 that biases the syringe 114 from an extended position in which the needle 118 extends from the aperture 128 in a case nose 112a of the housing 112 to a retracted position in which the needle 118 is contained within the housing 112. The return spring 126 acts on the syringe 114 via a syringe carrier 127. The syringe 114 is moveable along a longitudinal axis 105 of the injection device 110 which extends centrally along the length of the injection device 110 from the exit aperture 128 at its proximal end 167 to a distal end 168.

Contained within the housing at its distal end 168 is an actuator, which here takes the form of a compression drive spring 130. Drive from the drive spring 130 is transmitted via a multi-component drive 129 to the syringe 114 to advance it from its retracted position to its extended position and discharge its contents through the needle 118. The drive 129 accomplishes this task by acting directly on the drug and the syringe 114. Hydrostatic forces acting through the drug and, to a lesser extent, static friction between the bung 122 and the syringe body 116 initially ensure that they advance together, until the return spring 126 bottoms out on the syringe carrier 127 or meets some other obstruction (not shown) that retards its motion.

Fig. 2c is an exploded perspective view of the multi-component drive 129. The multi-component drive 129 between the drive spring 130 and the syringe 114 consists of three principal components. A drive sleeve 131 takes drive from the drive spring 130 and transmits it to a delay piston 133 on a first drive element 132. This in turn transmits drive to the second drive element 134.

As will be seen from Fig. 2c, the first drive element 132 includes a hollow stem 140, the inner cavity of which forms a collection chamber 141 in communication with a vent 144 that extends from the collection chamber 141 through the end of the stem 140. The second drive element 134 includes a blind bore 146 that is open at one end to receive the stem 140 and closed at the other. As will be appreciated, the bore 146 and the stem 140 define a fluid reservoir within which a damping fluid is contained.

The trigger button 102 is provided on the side of the housing 112 which, when in an engaged position with a proximal end 145 of the drive sleeve 131, serves to retain the drive spring 130 in a compressed state by contact between locking surface 102b and the drive sleeve 131 when the trigger button 102 is in an unactuated position. The trigger button 102 can pivot on the housing 112 via pivot 102a. When downwards pressure is applied to the trigger button 102 at an activation surface 102c (i.e. pressure directed into the housing 112), the locking surface 102b moves upwards in a direction away from the longitudinal axis 105. In this actuated position of the trigger button 102, the locking surface 102b is decoupled from the drive sleeve 131, thereby allowing the drive sleeve 131 to move relative to the housing 112 towards the exit aperture 128 under the influence of the drive spring 130.

The sliding sleeve 119 is moveable from its extended position (as shown in Fig. 1b) where it protrudes out of the exit aperture 128 into a retracted position in the case nose 112a of the housing 112. The sliding sleeve 119 is connected to a trigger button lock element 150 which has resilient arms 151 which bias the sliding sleeve 119 into its extended position in which its end protrudes from the end of the case nose 112a. Thus, application of pressure to the end of the sliding sleeve 119, for example by pressing the end of the sliding sleeve 119 against tissue, causes it to move into its retracted position into the housing 112; release of the pressure causes the sliding sleeve 119 to move into its extended position under bias from the resilient arms 151 acting against a side wall of the housing 112. The trigger button lock element 150 has a trigger button lock protrusion 152 which contacts with the end of a trigger button protrusion 102d on the trigger button 102 when the sliding sleeve is in its extended position. The trigger button protrusion 102 extends in a direction which is generally parallel to the longitudinal axis 105 of the injection device 110. The trigger button lock protrusion 152 extends in a direction which is generally perpendicular to the longitudinal axis 105 towards the trigger button protrusion 102d. The trigger button protrusion 102d has an aperture 102e which can move over the top of the trigger button lock protrusion 152 when the trigger button lock element 150 has been moved away from the exit aperture 128 (i.e. when the sliding sleeve 119 has been moved into the exit aperture 128 into its retracted position). In this position, the trigger button 102 can be moved into its deactivated position by rotating the trigger button 102 about the pivot 102a in the direction of the pressure applied to the pressure surface 102c. Thus, the trigger button lock element 150 and the sliding sleeve 119 act together to lock the trigger button 102 in its activated position (i.e. the locking surface 102b contacts the end of the drive sleeve 131 preventing it from moving towards the exit aperture 128 under the bias of the compressed drive spring 130).

When the sliding sleeve 119 has been moved into a position in which it is retracted into the housing 112 (i.e. into its unlocked position) and the trigger button 102 has been rotated into its deactivated position, the operation of the device 110 is then as follows.

Initially, the drive spring 130 moves the drive sleeve 131, the drive sleeve 131 moves the first drive element 132 and the first drive element 132 moves the second drive element 134, in each case by acting through flexible latch arms 132a, 134a, 134b. The second drive element 134 moves and, by virtue of static friction and hydrostatic forces acting through the drug (not shown), moves the syringe body 116 and syringe carrier 127 against the action of the return spring 126. The return spring 126 compresses and the hypodermic needle 118 emerges from the exit aperture 128 of the housing 112. This continues until the return spring 126 bottoms out or the syringe body 116 meets some other obstruction (not shown) that retards its motion. Because the static friction between the second drive element 134 and the syringe body 116 and the hydrostatic forces acting through the drug (not shown) to be administered are not sufficient to resist the full drive force developed by the drive spring 130, at this point the second drive element 134 begins to move within the syringe body 116 and the drug (not shown) begins to be discharged. Dynamic friction between the second drive element 134 and the syringe body 116 and hydrostatic forces acting through the drug (not shown) to be administered are, however, sufficient to retain the return spring 126 in its compressed state, so the hypodermic needle 118 remains extended.

Before the second drive element 134 reaches the end of its travel within the syringe body 116, so before the contents of the syringe have fully discharged, the flexible latch arms 134a, 134b linking the first and second drive elements 132, 134 reach a constriction 137 provided on a latch actuator element 137a which is fixed to the end of the syringe carrier 127. The constriction 137 moves the flexible latch arms 134a, 134b inwards from the position shown in Fig. 2c to a position at which the flexible latch arms 134a, 134b no longer couple the first drive element 132 to the second drive element 134, aided by the bevelled surfaces on the constriction 137. Once this happens, the first drive element 132 acts no longer on the second drive element 134, allowing the first drive element 132 to move relative to the second drive element 134.

Because the damping fluid is contained within a reservoir (not shown) defined between the end of the first drive element 132 and the blind bore 146 in the second drive element 134, the volume of the reservoir will tend to decrease as the first drive element 132 moves relative to the second drive element 134 when the former is acted upon by the drive spring 130. As the reservoir collapses, damping fluid is forced through the vent 144 into the collection chamber 141. Thus, once the flexible latch arms 134a, 134b have been released, the force exerted by the drive spring 130 does work on the damping fluid, causing it to flow though the constriction formed by the vent 144, and also acts hydrostatically through the fluid and through friction between the first and second drive elements 132, 134, thence via the second drive element 134. Losses associated with the flow of the damping fluid do not attenuate the force acting on the body of the syringe to a great extent. Thus, the return spring 126 remains compressed and the hypodermic needle remains extended.

After a time, the second drive element 134 completes its travel within the syringe body 116 and can go no further. At this point, the contents of the syringe 114 are completely discharged and the force exerted by the drive spring 130 acts to retain the second drive element 134 in its terminal position and to continue to cause the damping fluid to flow though the vent 144, allowing the first drive element 132 to continue its movement.

Before the reservoir of fluid is exhausted, the flexible latch arms 132a linking the drive sleeve 131 with the first drive element 132 reach another constriction (not shown) within the housing 112. This constriction moves the flexible latch arms 132a inwards from the position shown to a position at which they no longer couple the drive sleeve 131 to the first drive element 132, aided by bevelled surfaces on the constriction. Once this happens, the drive sleeve 131 acts no longer on the first drive element 132, allowing them to move relative each other. At this point, of course, the syringe 114 is released, because the forces developed by the drive spring 130 are no longer being transmitted to the syringe 114, and the only force acting on the syringe will be the return force from the return spring 126. Thus, the syringe 114 is now returned to its retracted position and the injection cycle is complete.

All this takes place, of course, only once the cap 111 has been removed from the end of the housing 112. The end of the syringe is sealed with a boot 123. The central boss 121 of the cap 111 that fits within the sleeve 119 when the cap 111 is installed on the housing 112 comprises a retainer element 125 which is fixed into the boss 121. The retainer element 125 comprises resilient protrusions 125a which are directed away from the exit aperture 128. These resilient protrusions 125a deform as the cap 111 is inserted onto the housing 112 over a needle shield or rubber boot 123. The protrusions 125a then grip the boot 123 tightly so that the ends of the protrusions are slightly embedded in the boot 123 which might be made from rubber. This means that, as the cap 111 is pulled off the housing 112, the boot 123 is pulled away from the syringe 114 with the cap 111.

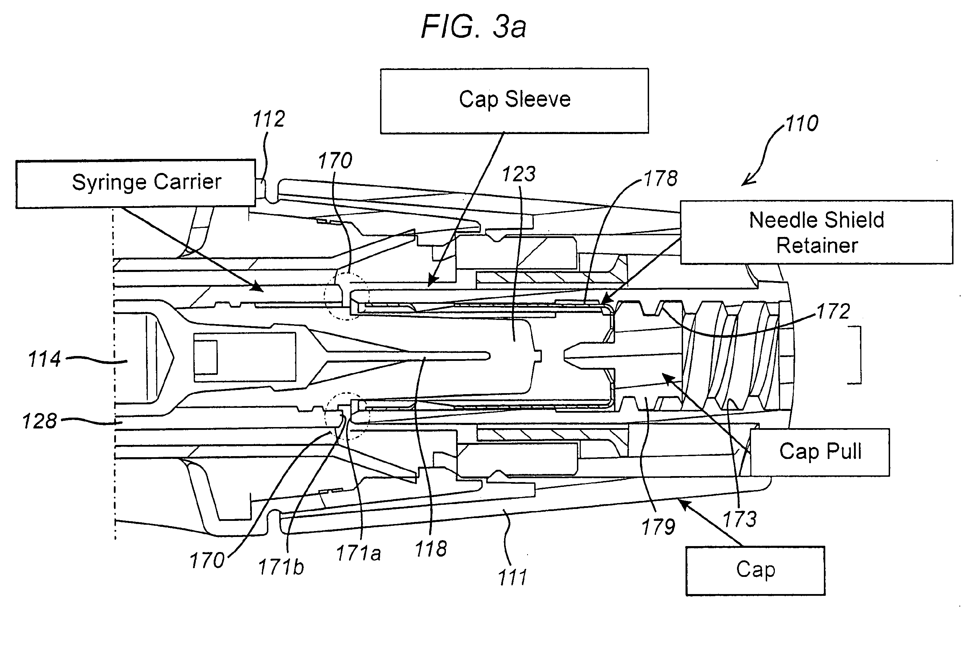

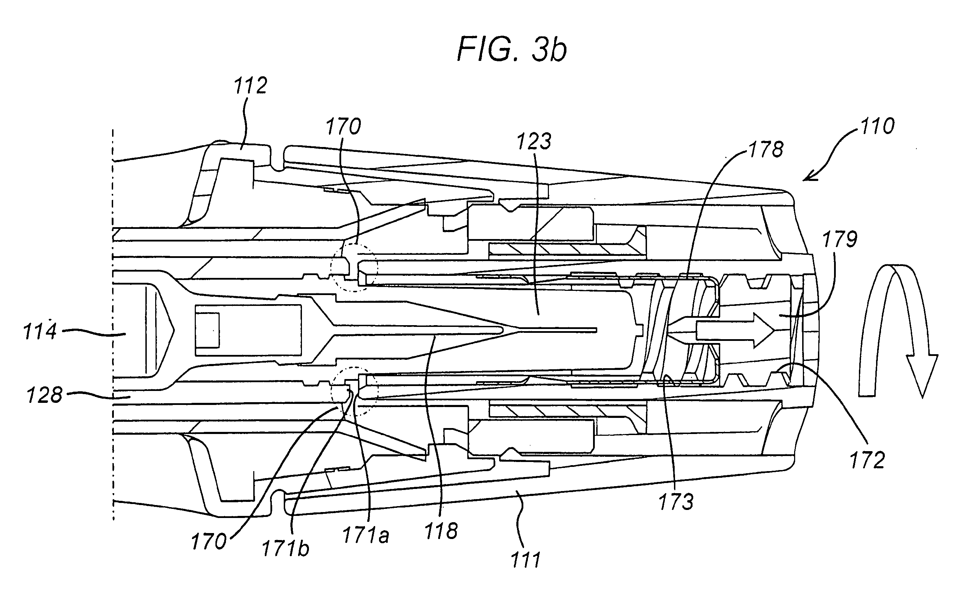

Figs. 3a and 3b show a locking mechanism 170 for the syringe carrier 127 included in the injection device 110.

The locking mechanism 170 comprises a locking component 171 which is an abutment surface 171a on the end of the central boss 121 (or sleeve) which is adapted to contact a second abutment surface 171 b located the end of the syringe carrier 128 to prevent the syringe carrier 127 moving towards the proximal end 167 of the injection device 110, i.e. towards the exit aperture 128, when the cap is located in its closed position over the exit aperture 128.

With the cap 111 in its closed position, the shield retainer 125 is moveable within the cap 111 in a linear direction along the longitudinal axis 105 away from the exit aperture 128 to release the needle shield 123 from the discharge nozzle 118. This is achieved by rotation of the cap 111 about the longitudinal axis 105 whilst the cap is still in its closed position (i.e. covering the exit aperture 128).

The shield retainer 125 is provided with a shield grip 178 and a shield pull component 179 connected to the shield grip 178. A first screw thread 172 is provided on outside of the shield pull component 179 which engages with a second screw thread 173 provided on the inside of central boss 121.

During rotation, the shield retainer 125 does not rotate relative to the discharge nozzle 118 and the rotational movement of the cap 111 relative to the housing 112 is converted into linear movement of the shield retainer 125 away from the exit aperture 128 along the longitudinal axis 105 by the screw threads 172, 173. This conversion of rotational motion to linear motion is achieved through engagement of screw threads 172, 173 on the central boss 121 and on the shield retainer 125. Since the abutment surface 171 a is in contact with the second abutment surface 171 b, the syringe carrier 128 is prevented from moving towards the proximal end 167 of the injection device 110, so the needle shield 123 is pulled away from the discharge nozzle 118 through the exit aperture 128. into the central boss 121. Thus, after rotation, when the cap 111 is finally removed by a user of the injection device 110 by pulling it away from the housing 112, the needle shield 123 and discharge nozzle 118 are not engaged with each other and the cap becomes completely detached from the injection device 110 without pulling the syringe carrier 128 forward.

Thus, the cap 111 prevents movement of the syringe carrier 127 towards the exit aperture relative to the housing 112 when the cap 111 is located on the housing 112, but still permits the needle shield 123 to be removed from the discharge nozzle 118 by rotation of the cap 111. Thus, the syringe carrier 128 cannot move linearly when the cap 111 is in place and the needle shield 123 is being removed by rotation of the cap 111.

Fig. 3a shows the needle shield 123 located over the discharge nozzle 118 prior to rotation of the cap 111. Fig. 3b shows the needle shield 123 having been released from the discharge nozzle 118 during rotation of the cap 111.

It will of course be understood that the present invention has been described above purely by way of example and modifications of detail can be made within the scope of the claims.

## Claims

1. An injection device (110) comprising:
a housing (112) adapted to receive a syringe (114) having a discharge nozzle (118), the syringe being movable along a longitudinal axis of the housing between a retracted position in which the discharge nozzle is contained within the housing and an extended position in which the discharge nozzle extends from the housing through an exit aperture (128);
a cap (111) removably located over the exit aperture;
a syringe carrier (127) adapted to support the syringe as it is advanced; and
a needle shield (123) over the discharge nozzle,
wherein the needle shield is connected to the cap,
wherein the cap comprises a locking component (171) which is adapted to prevent, in an engaged position when the cap is located on the housing, movement of the syringe carrier towards the exit aperture relative to the housing as the needle shield is removed from the discharge nozzle, **characterised in that**
the needle shield (127) is moveable within the cap (111) in a linear direction towards the exit aperture to release the needle shield from the discharge nozzle.

2. The injection device of claim 1, wherein the cap is rotatable on the injection device to move the needle shield in its linear direction towards the exit aperture.

3. An injection device according to claim 1 or claim 2, wherein the cap comprises a body and the locking component is a sleeve (121) located within the body and fixed relative to the body.

4. The injection device of claim 3, wherein the cap further comprises a shield retainer (125) adapted to grip the needle shield.

5. An injection device according to claim 4, wherein the body of the cap is rotatable relative to the housing whilst the needle shield does not rotate relative to the discharge nozzle, wherein the cap comprises means for converting rotational movement of the cap relative to the housing into linear movement of the needle shield away from the exit aperture along the longitudinal axis.

6. An injection device according to claim 5, wherein the shield retainer is located within the sleeve.

7. An injection device according to claim 5 or claim 6, wherein the converting means is provided by a first thread (172) on the shield retainer and a second thread (173) on the sleeve and the first and second threads engage each other so that rotational movement of the body of the cap results in linear movement of the shield retainer with respect to the exit aperture.

8. An injection device according to claim 7, wherein the shield retainer is provided with a shield grip (178) and a shield pull component (179) connected to the shield grip, wherein the first thread is provided on the shield pull component.

9. An injection device according to any one of claims 5 to 8, wherein the rotational movement is rotation about the longitudinal axis.

10. An injection device according to any one of the preceding claims, wherein the sleeve comprises a first abutment surface (171a) adapted to abut a second abutment surface (171b) on the syringe carrier when the cap is located over the exit aperture, thereby preventing movement of the syringe carrier along the longitudinal axis towards the exit aperture.

11. A method of removing a cap (111) from an injection device (110) having a housing (112) and a syringe (114) located in the housing, the syringe moveable along a longitudinal axis of the housing between a retracted position in which the discharge nozzle is contained within the housing and an extended position in which the discharge nozzle extends from the housing through an exit aperture (128), the cap gripping a needle shield (123) of the syringe, the method comprising:
rotating the cap about the longitudinal axis such that the needle shield is moved along the longitudinal axis out of the exit aperture, thereby releasing it from the discharge nozzle whilst a locking component (171) in the cap prevents movement of the syringe along the longitudinal axis; and
when the needle shield has been released from the discharge nozzle, moving the cap along the longitudinal axis to expose the exit aperture and release the locking component so that the discharge nozzle can be moved to its extended position.

## Patentansprüche

1. Injektionsvorrichtung (110), umfassend:
ein Gehäuse (112) zur Aufnahme einer Spritze (114) mit einer Ausgabedüse (118), wobei die Spritze entlang einer Längsachse des Gehäuses zwischen einer zurückgezogenen Stellung, in der die Ausgabedüse im Gehäuse enthalten ist, und einer ausgefahrenen Stellung, in der sich die Ausgabedüse durch eine Austrittsöffnung (128) vom Gehäuse erstreckt, bewegt werden kann;
eine Kappe (111), die abnehmbar über der Austrittsöffnung angeordnet ist;
einen Spritzenträger (127) zur Unterstützung der Spritze, wenn diese vorgeschoben wird; und
einen Nadelschutz (123) über der Ausgabedüse,
worin der Nadelschutz mit der Kappe verbunden ist,
worin die Kappe eine Sperrkomponente (171) umfasst, die in einer Eingriffsstellung, wenn sich die Kappe auf dem Gehäuse befindet, eine Bewegung des Spritzenträgers zur Austrittsöffnung in Bezug auf das Gehäuse verhindern kann, wenn der Nadelschutz von der Ausgabedüse entfernt wird, **dadurch gekennzeichnet, dass**
der Nadelschutz (127) in der Kappe (111) zur Freisetzung des Nadelschutzes aus der Ausgabedüse in einer linearen Richtung zur Austrittsöffnung bewegt werden kann.

2. Injektionsvorrichtung nach Anspruch 1, worin die Kappe auf der Injektionsvorrichtung drehbar ist, um den Nadelschutz in seiner linearen Richtung zur Austrittsöffnung zu bewegen.

3. Injektionsvorrichtung nach Anspruch 1 oder Anspruch 2, worin die Kappe einen Körper umfasst und die Sperrkomponente eine Hülse (121) ist, die im Körper angeordnet ist und bezüglich des Körpers fixiert ist.

4. Injektionsvorrichtung nach Anspruch 3, worin die Kappe ferner eine Nadelschutzhalteeinrichtung (125) zum Greifen des Nadelschutzes umfasst.

5. Injektionsvorrichtung nach Anspruch 4, worin der Körper der Kappe bezüglich des Gehäuses drehbar ist, während der Nadelschutz sich relativ zur Ausgabedüse nicht dreht, worin die Kappe ein Mittel zur Umwandlung der Drehbewegung der Kappe bezüglich des Gehäuses in eine lineare Bewegung des Nadelschutzes von der Austrittsöffnung weg entlang der Längsachse umfasst.

6. Injektionsvorrichtung nach Anspruch 5, worin die Nadelschutzhalteeinrichtung in der Hülse angeordnet ist.

7. Injektionsvorrichtung nach Anspruch 5 oder Anspruch 6, worin das Umwandlungsmittel von einem ersten Gewinde (172) auf der Nadelschutzrückhalteeinrichtung und einem zweiten Gewinde (173) auf der Hülse bereitgestellt wird und die ersten und zweiten Gewinde ineinander greifen, so dass eine Drehbewegung des Körpers der Kappe zu einer linearen Bewegung der Nadelschutzrückhalteeinrichtung in Beziehung auf die Austrittsöffnung führt.

8. Injektionsvorrichtung nach Anspruch 7, worin die Nadelschutzrückhalteeinrichtung mit einem Nadelschutzgriff (178) und einer mit dem Nadelschutzgriff verbundenen Nadelschutzzugkomponente (179) versehen ist, worin das erste Gewinde auf der Nadelschutzzugkomponente vorgesehen ist.

9. Injektionsvorrichtung nach einem der Ansprüche 5 bis 8, worin die Drehbewegung eine Drehung um die Längsachse ist.

10. Injektionsvorrichtung nach einem der vorhergehenden Ansprüche, worin die Hülse eine erste Anlagefläche (171a) umfasst, die an einer zweiten Anlagefläche (171b) auf dem Spritzenträger anliegen kann, wenn sich die Kappe über der Austrittsöffnung befindet, wodurch eine Bewegung des Spritzenträgers entlang der Längsachse zur Austrittsöffnung hin verhindert wird.

11. Verfahren zur Entfernung einer Kappe (111) von einer Injektionsvorrichtung (110) mit einem Gehäuse (112) und einer im Gehäuse angeordneten Spritze (114), wobei die Spritze entlang einer Längsachse des Gehäuses zwischen einer zurückgezogenen Stellung, in der die Ausgabedüse im Gehäuse enthalten ist, und einer ausgefahrenen Stellung, in der sich die Ausgabedüse durch eine Austrittsöffnung (128) vom Gehäuse erstreckt, bewegt werden kann, wobei die Kappe einen Nadelschutz (123) der Spritze greift, wobei das Verfahren Folgendes umfasst:
Drehung der Kappe um die Längsachse, so dass der Nadelschutz entlang der Längsachse und aus der Austrittsöffnung bewegt wird, wodurch sie aus der Ausgabedüse freigesetzt wird, während eine Sperrkomponente (171) in der Kappe eine Bewegung der Spritze entlang der Längsachse verhindert; und
nach Freisetzung des Nadelschutzes aus der Ausgabedüse Bewegung der Kappe entlang der Längsachse zum Freilegen der Austrittsöffnung und zur Freisetzung der Sperrkomponente, so dass die Ausgabedüse in ihre ausgefahrene Stellung bewegt werden kann.

## Revendications

1. Dispositif (110) d'injection comportant :
un logement (112) conçu de manière à recevoir une seringue (114) munie d'une buse (118) d'évacuation, la seringue étant mobile le long d'un axe longitudinal du logement entre une position de retrait dans laquelle la buse d'évacuation est contenue à l'intérieur du logement et une position d'extension dans laquelle la buse d'évacuation s'étend depuis le logement à travers une ouverture (128) de sortie ;
un capuchon (111) situé de manière amovible sur l'ouverture de sortie ;
un porte-seringue (127) conçu de manière à supporter la seringue au fur et à mesure qu'elle est avancée ; et
une protection (123) d'aiguille sur la buse d'évacuation,
dans lequel la protection d'aiguille est reliée au capuchon,
dans lequel le capuchon comporte un élément (171) de verrouillage qui est conçu de manière à empêcher, dans une position de mise en prise lorsque le capuchon est situé sur le logement, le déplacement du porte-seringue vers l'ouverture de sortie par rapport au logement au fur et à mesure que la protection d'aiguille est enlevée de la buse d'évacuation, **caractérisé en ce que**
la protection (127) d'aiguille est mobile à l'intérieur du capuchon (111) dans une direction linéaire vers l'ouverture de sortie pour libérer la protection d'aiguille de la buse d'évacuation.

2. Dispositif d'injection selon la revendication 1, dans lequel le capuchon est de rotation sur le dispositif d'injection de manière à déplacer la protection d'aiguille dans sa direction linéaire vers l'ouverture de sortie.

3. Dispositif d'injection selon la revendication 1 ou la revendication 2, dans lequel le capuchon comporte un corps et le composant de verrouillage est un manchon (121) situé à l'intérieur du corps et fixe par rapport au corps.

4. Dispositif d'injection selon la revendication 3, dans lequel le capuchon comporte en outre un élément de retenue (125) de la protection conçu de manière à saisir la protection d'aiguille.

5. Dispositif d'injection selon la revendication 4, dans lequel le corps du capuchon tourne par rapport au logement tandis que la protection d'aiguille ne tourne pas par rapport à la buse d'évacuation, dans lequel le capuchon comporte un moyen destiné à transformer le mouvement de rotation du capuchon par rapport au logement en un mouvement linéaire de la protection d'aiguille dans le sens s'éloignant de l'ouverture de sortie le long de l'axe longitudinal.

6. Dispositif d'injection selon la revendication 5, dans lequel l'élément de retenue de la protection est situé à l'intérieur du manchon.

7. Dispositif d'injection selon la revendication 5 ou la revendication 6, dans lequel le moyen de transformation du mouvement est fourni par un premier filet (172) sur l'élément de retenue de la protection et un second filet (173) sur le manchon et les premier et second filets sont en prise l'un avec l'autre de manière à ce que le mouvement de rotation du corps du capuchon se transforme en un mouvement linéaire de l'élément de retenue de la protection par rapport à l'ouverture de sortie.

8. Dispositif d'injection selon la revendication 7, dans lequel l'élément de retenue de la protection est fourni avec un élément de saisie (178) de la protection et un composant (179) de traction de la protection relié à l'élément de saisie de la protection, le premier filet étant fourni sur le composant de traction de la protection.

9. Dispositif d'injection selon l'une quelconque des revendications 5 à 8, dans lequel le mouvement de rotation représente une rotation autour de l'axe longitudinal.

10. Dispositif d'injection selon l'une quelconque des revendications précédentes, dans lequel le manchon comporte une première surface (171a) de butée conçue de manière à buter contre une seconde surface (171b) de butée sur le porte-seringue lorsque le capuchon est situé sur l'ouverture de sortie, empêchant ainsi le déplacement du porte-seringue le long de l'axe longitudinal vers l'ouverture de sortie.

11. Procédé d'enlèvement d'un capuchon (111) d'un dispositif (110) d'injection muni d'un logement (112) et d'une seringue (114) située dans le logement, la seringue étant mobile le long d'un axe longitudinal du logement entre une position de retrait dans laquelle la buse d'évacuation est contenue à l'intérieur du logement et une position d'extension dans laquelle la buse d'évacuation s'étend du logement à travers une ouverture (128) de sortie, le capuchon saisissant une protection (123) d'aiguille de la seringue, le procédé consistant à :
faire tourner le capuchon autour de l'axe longitudinal de telle sorte que la protection d'aiguille est déplacée le long de l'axe longitudinal hors de l'ouverture de sortie, la libérant ainsi de la buse d'évacuation tandis que le composant (171) de verrouillage dans le capuchon empêche le déplacement de la seringue le long de l'axe longitudinal ; et
lorsque la protection d'aiguille a été libérée de la buse d'évacuation, déplacer le capuchon le long de l'axe longitudinal pour exposer l'ouverture de sortie et libérer le composant de verrouillage de façon à ce que la buse d'évacuation puisse être déplacée vers sa position d'extension.
